# EUROPEAN PATENT SPECIFICATION

(11) **EP 1 729 727 B1**
(45) Date of publication and mention of the grant of the patent: **03.07.2019**
(21) Application number: 05767549.8
(22) Date of filing: 01.04.2005
(51) Int. Cl.: A61K 9/28, A61K 31/52

(54) **IMPROVED FORMULATIONS OF 6-MERCAPTOPURINE**
VERBESSERTE FORMULIERUNGEN DES 6-MERCAPTOPURINS
FORMULATIONS AMELIOREES DE 6-MERCAPTOPURINE

(30) Priority: 01.04.2004 US 558477 P
(43) Date of publication of application: 13.12.2006
(73) Proprietor: Hadasit Medical Research Services and Development Ltd., 9112001 Jerusalem (IL)
(72) Inventor: LERNER, Itzhak, E., Petach Tikva 49404 (IL); FLASHNER-BARAK, Moshe, 49313 Petach Tikva (IL); ACHTHOVEN, Erwin v, 2353 EM Leiderdorp (NL); KEEGSTRA, Hans, 1816 CR Alkmaar (NL); SMIT, Ruud, 2026 TJ Haarlem (NL)
(74) Representative: D Young & Co LLP
(86) International application number: PCT/US2005/011112
(87) International publication number: WO 2005/099665

(56) References cited:
- TAKEICHI YOH'ICHIRO ET AL: "Improvement of aqueous solubility and rectal absorption of 6-mercaptopurine by addition of sodium benzoate" BIOLOGICAL AND PHARMACEUTICAL BULLETIN, vol. 17, no. 10, 1994, pages 1391-1394, XP002402581 ISSN: 0918-6158
- GLAXOSMITHKLINE: "Purinethol Prescribing Information" INTERNET, [Online] 2002, XP002402582 Retrieved from the Internet: URL:http://us.gsk.com/products/assets/us_p urinethol.pdf> [retrieved on 2006-10-11]
- "Physician's Desk Reference 57th Edition", 2003 pages 1615-1616,

## Description

### FIELD OF THE INVENTION

The present invention relates to a process for preparing improved formulations of 6-mercaptopurine as well as pharmaceutical compositions comprising the improved formulations of 6-mercaptopurine where the improved formulations exhibit a faster release of 6-mercaptopurine under aqueous conditions than prior art formulations and exhibit more favorable bioavailability profiles than prior art formulations.

### BACKGROUND OF THE INVENTION

6-mercaptopurine (6-MP) is a synthetic analogue of natural purine bases. After absorption into the body, it is transformed into nucleotides which interfere with nucleic acid biosynthesis, especially in the active S phase. As such, it used to slow the growth of cancerous cells. 6-MP is indicated as a monotherapy and as part of combination therapies for treating acute lymphocytic leukemia in both adults and children (Physician's Desk Reference 57th Edition, 2003, page 1615-1618). 6-MP also exhibits immunosuppressive properties. While it is not officially indicated for diseases where treatment with immunosuppressive agents is beneficial, 6-MP has been widely used for several such conditions, especially for Crohn's disease and colitis.

6-MP is administered orally and has partial and variable absorption and bioavailability. Approximately 50% of an oral dose is absorbed. 6-MP is further subject to metabolism, especially by thiopurine methyltransferase.

The need for improving the therapeutic potential of 6-MP has been known for a long time. U.S. Patents Nos. 4,443,435 and 5,120,740, among others, describe the preparation of prodrugs for 6-MP as ways of improving the use of this potent drug. Work of this sort continues, as is seen in U.S. Patent Application Publications 20040013728, 20030232760, and 20020013287. U.S. Patents Nos. 6,680,302; 6,576,438; and 6,355,623 describe methods of improving the therapeutic outcome of 6-MP treatment in leukemia and in bowel diseases such as Crohn's disease or colitis by monitoring metabolites of the 6-MP and/or thiopurine methyltransferase activity and setting dosing based on the results. U.S. Patents Nos. 6,692,771 and 6,680,068 and U.S. Patent Application Publications 20030077306 and 20020160049 describe emulsion formulations that may help the penetration of 6-MP into the body, while U.S. Patents Nos. 6,602,521 and 6,372,254, and U.S. Patent Application Publications 20030133976 and 20020164371 describe drug delivery systems that might improve the therapeutics of 6-MP. None of these latter patents show data demonstrating improved bioavailability or therapeutic outcomes with 6-MP. The need still exists for formulations for improved delivery of 6-MP that improve the bioavailability thereof.

Standard 6-MP tablets (described in Physician's Desk Reference 57th Edition, 2003, page 1615-1618) reach full dissolution after about an hour under acidic dissolution conditions using a USP type II dissolution unit with paddles rotating at 50 rpm. 50% dissolution is reached at between 10 and 15 minutes. This rate of dissolution is not as fast as would be desirable. One method of improving the rate of dissolution of poorly soluble powders is to micronize them. In the case of 6-MP, micronization does little to improve the rate of dissolution of formulated tablets when compared to the standard formulation. The lack of improved rate of dissolution makes such tablets unlikely to show improved bioavailability when compared to the standard formulation. Further improvements to the formulation are clearly needed.

Takeichi Yoh'ichiro et al. ("Improvement of aqueous solubility and rectal absorption of 6-mercaptopurine by addition of sodium benzoate", Biological and Pharmaceutical Bulletin, vol. 17, no. 10, 1994, pages 1391-1394) discloses improving aqueous solubility and rectal absorption of 6-mercaptopurine by addition of sodium benzoate.

GlaxoSmithKline ("Purinethol Prescribing Information", 2002, Retrieved from the Internet: URL: http://us.gsk.com/products/assets/us_purinethol.pdf [retrieved on 2006-10-11]) discloses prescribing information for a commercially available formulation of 6-mercaptopurine.

### SUMMARY OF INVENTION

The present invention is directed to compositions of 6-mercaptopurine which give improved rates of dissolution when tested in a dissolution bath. It has been found that by granulating solutions of 6-mercaptopurine and pharmaceutical carriers, and forming tablets therefrom, compositions are produced that improve the rate of dissolution of the 6-mercaptopurine. It has been further found that improvement in the rate of dissolution of the 6-mercaptopurine leads to an improvement in the bioavailability of the 6-mercaptopurine.

In one aspect, the invention relates to a pharmaceutical composition comprising a pharmaceutical carrier powder, 6-mercaptopurine, a pharmaceutically acceptable base, and a pharmaceutically acceptable acid selected from the group consisting of acetic acid, ascorbic acid, citric acid, and tartaric acid, wherein the pharmaceutical composition exhibits enhanced solubility in aqueous acid as compared to the standard formulation as defined in the Physician's Desk Reference 57th Edition, 2003, page 1615-1618 and wherein a solution of the pharmaceutically acceptable acid is sprayed onto the pharmaceutical carrier powder in a molar amount that is greater than the molar amount of pharmaceutically acceptable base and a solution of the 6-mercaptopurine and the pharmaceutically acceptable base is spray granulated onto the pharmaceutical carrier powder over the pharmaceutically acceptable acid.

In another aspect, the invention relates to a pharmaceutical composition as described above for use in the treatment of Crohn's disease, which provides to the subject a therapeutic outcome with an improved side effect profile as compared to treating the subject with the standard formulation of 6-mercaptopurine as defined in the Physician's Desk Reference 57th Edition, 2003, page 1615-1618, wherein the treatment comprises orally administering to the subject the pharmaceutical composition of the invention.

In one embodiment of the pharmaceutical composition, the spray granulation was carried out in a fluidized bed.

In another embodiment of the pharmaceutical composition, the solution of the 6-mercaptopurine and the pharmaceutically acceptable base comprises:
a solvent selected from the group consisting of: water and an at least about stoichiometric amount of a pharmaceutically acceptable base, ethanol and an at least about stoichiometric amount of a pharmaceutically acceptable base, and ethanol/water mixtures and an at least about stoichiometric amount of a pharmaceutically acceptable base.

In another embodiment of the pharmaceutical composition, the solvent for the solution of the 6-mercaptopurine and the pharmaceutically acceptable base comprises ethanol/water/potassium hydroxide, ethanol/water/sodium hydroxide, or ethanol/potassium hydroxide.

In another embodiment of the pharmaceutical composition, the pharmaceutically acceptable acid is citric acid.

In one embodiment of the pharmaceutical composition for use, the pharmaceutical composition comprises 0.5 to 150 mg of 6-mercaptopurine, preferably the pharmaceutical composition comprises 50 mg of 6-mercaptopurine.

In another embodiment of the pharmaceutical composition for use, the pharmaceutical composition contains 15% less 6-mercaptopurine than the amount of 6-mercaptopurine in the standard formulation of 6-mercaptopurine as defined in the Physician's Desk Reference 57th Edition, 2003, page 1615-1618.

In another aspect, the invention relates to a pharmaceutical composition comprising a pharmaceutical carrier powder and 6-mercaptopurine, wherein the pharmaceutical composition exhibits enhanced solubility in aqueous acid as compared to the standard formulation as defined in the Physician's Desk Reference 57th Edition, 2003, page 1615-1618 and wherein a solution of the 6-mercaptopurine and an organic solvent is spray granulated onto the pharmaceutical carrier powder.

In another embodiment of the pharmaceutical composition, the pharmaceutical carrier powder comprises a powder selected from the group consisting of: lactose, starch, microcrystalline cellulose, calcium phosphate, powdered cellulose, sorbitol, and sucrose, preferably the powder is lactose or microcrystalline cellulose.

In some embodiments of the pharmaceutical composition, the organic solvent is selected from the group consisting of dimethylformamide, dimethylacetamide, dimethylsulfoxide, and mixtures thereof.

In another aspect, the invention relates to a method of manufacturing a pharmaceutical composition comprising:
(a) spraying a solution of a pharmaceutically acceptable acid onto a pharmaceutical carrier;
(b) spray granulating the pharmaceutical carrier sprayed with a pharmaceutically acceptable acid produced in step (a) with a solution of 6-mercaptopurine and a pharmaceutically acceptable base, wherein the molar amount of the pharmaceutically acceptable acid is greater than the molar amount of the pharmaceutically acceptable base.

In one embodiment, of the method of manufacturing a pharmaceutical composition the pharmaceutical carrier comprises a powder selected from the group consisting of lactose, starch, microcrystalline cellulose, calcium phosphate, powdered cellulose, sorbitol, and sucrose, preferably the powder is lactose or microcrystalline cellulose.

In another embodiment of the method of manufacturing a pharmaceutical composition, the pharmaceutically acceptable acid is selected from the group consisting of: acetic acid, ascorbic acid, citric acid and tartaric acid, preferably the pharmaceutically acceptable acid is citric acid.

In another embodiment of the method of manufacturing a pharmaceutical composition the pharmaceutically acceptable base is selected from the group consisting of: hydroxide or carbonate salts of potassium, sodium, magnesium, ammonium or calcium, preferably the pharmaceutically acceptable base is potassium hydroxide.

In another embodiment of the method of manufacturing a pharmaceutical composition the 6-mercaptopurine forms a coating on the solid carrier, preferably the 6-mercaptopurine forms a uniform coating on the solid carrier.

### DESCRIPTION OF THE DRAWINGS

Figure 1 shows the dissolution of a 6-mercaptopurine composition of the present invention (6-MP-IB) versus PURINETHOL® in 0.1 N HCl (see Example 1).
Figure 2 shows the dissolution of a 6-mercaptopurine composition of the present invention from (6-MP-IB batch) vs. PURINETHOL® in 0.1N HCl (see Example 2).
Figure 3 shows the average pharmacokinetic profile of 6-mercaptopurine for a pharmaceutical composition of the present invention (6-MP-IB batch) vs. the standard formulation (PURINETHOL®) (see Example 4).
Figure 4 shows the dissolution of a 6-mercaptopurine tablets prepared as in Example 3. - ▲- = PURINETHOL®; -◆- = tablets prepared with microcrystalline cellulose; -■- = tablets prepared with lactose; -x- = lactose tablets, 70% ethanol, 30% water, n=3 (average of three tablets).

### DESCRIPTION OF THE PREFERRED EMBODIMENTS

The present invention is directed to compositions of 6-mercaptopurine which give improved rate of dissolution when tested in a dissolution bath and show improved bioavailability characteristics when dosed to mammals.

As used herein, the "standard formulation" is the formulation described in the Physician's Desk Reference, 57th edition, 2003, pages 1615-1618 and sold in the United States under the brand name PURINETHOL®.

As used herein, the term "enhanced solubility properties" or "enhanced solubility" of a material or composition of the present invention means an improved rate of dissolution of the material or composition of the present invention or an improved extent of dissolution of the material or composition of the present invention as compared to the standard formulation.

As used herein, the term "improved bioavailability" refers to the increase in concentration of a drug in the body fluid provided by the compositions of the present invention as compared to the concentration of the drug in the body fluid from the standard formulation under identical conditions. Drug bioavailability is proportional to, and is typically measured by, the total area under the curve (AUC) of the concentration of the drug found in blood or plasma versus time when measured in a pharmacokinetic trial in a human or an animal. The AUC may be expressed as AUCt, *i.e.* the area under the curve to the last measured time point, or AUC_{I}, *i.e.* the area under the curve extrapolated to infinite time. The improvement in bioavailability is measured by the percent increase in the average AUC of the subjects in the trial when dosing the improved formulation as compared to the average AUC of the same subjects obtained by dosing of the standard formulation of the drug. Alternatively, the AUC ratio of the test formulation (AUCf) to the AUC of the reference formulation (AUCr) may be calculated on a per subject basis and then averaged. A percent of the average ratio (AUCf/AUCr) above 100% is then the improvement in bioavailability.

As used herein, the term "slight stoichiometric excess" refers to a stoichiometric excess of about 0.1% to about 30%, preferably about 0.5% to about 15%, more preferably about 1% to about 5%, in terms of mole percent.

As used herein, "pre-sprayed" refers to spraying the pharmaceutical carrier powder with the acid before the acid-sprayed pharmaceutical carrier is contacted with the solution of 6-mercaptopurine.

As used herein, "powder" in reference to a pharmaceutical carrier refers to particles of the pharmaceutical carrier having a size range of 1 to 800 microns, more preferably 2 to 500 microns, and most preferably 2 to 100 microns or 50 to 400 microns, depending on the material.

Described herein are compositions of 6-mercaptopurine which give improved rates of dissolution when tested in a dissolution bath. It has been found that by granulating solutions of 6-mercaptopurine and pharmaceutical carriers, and forming tablets therefrom, compositions are produced that improve the rate of dissolution of the 6-mercaptopurine. It has been further found that improvement in the rate of dissolution of the 6-mercaptopurine leads to an improvement in the bioavailability of the 6-mercaptopurine.

The present disclosure includes pharmaceutical compositions comprising 6-mercaptopurine wherein the dissolution of the 6-mercaptopurine is greater than 50% within seven minutes when the dissolution of a tablet comprising 50 mg of 6-mercaptopurine is measured in 900 ml of 0.1N HCl at 37°C in a USP type II device using paddles rotating at 50 rpm.

The present disclosure includes pharmaceutical compositions comprising 6-mercaptopurine wherein the time to reach 50% dissolution of the 6-mercaptopurine is reduced by at least about 30% compared to the standard formulation when the dissolution of a tablet comprising the pharmaceutical composition comprising 6-mercaptopurine is measured in 900 ml of 0.1N HCl at 37°C in a USP type II device using paddles rotating at 50 rpm.

The present disclosure includes pharmaceutical compositions comprising 6-mercaptopurine wherein the bioavailability is improved by at least about 15% when dosed to a mammal as compared to the standard formulation.

In one embodiment, the 6-mercaptopurine from the pharmaceutical composition comprising 6-mercaptopurine was spray granulated from a solution onto an acceptable pharmaceutical carrier powder.

In another embodiment, the 6-mercaptopurine from the pharmaceutical composition comprising 6-mercaptopurine was spray granulated from a solution onto an acceptable pharmaceutical carrier powder wherein the spray granulation was carried out in a fluidized bed.

In another embodiment, the 6-mercaptopurine from the pharmaceutical composition comprising 6-mercaptopurine was spray granulated from a solution onto an acceptable pharmaceutical carrier powder wherein the solvent for the solution of 6-mercaptopurine comprises a solvent selected from the group consisting of dimethylformamide, dimethylacetamide, dimethylsulfoxide, and mixtures thereof.

In another embodiment the 6-mercaptopurine from the pharmaceutical composition comprising 6-mercaptopurine was spray granulated from a solution onto an acceptable pharmaceutical carrier powder wherein the solvent for the solution of 6-mercaptopurine comprises a solvent selected from the group consisting of water and an at least about stoichiometric amount of a pharmaceutically acceptable base, ethanol and an at least about stoichiometric amount of a pharmaceutically acceptable base, and ethanol/water mixtures and an at least about stoichiometric amount of a pharmaceutically acceptable base.

In another embodiment, the 6-mercaptopurine from the pharmaceutical composition comprising 6-mercaptopurine was spray granulated from a solution onto an acceptable pharmaceutical carrier powder wherein the solvent for the solution of 6-mercaptopurine comprises a solvent selected from the group consisting of ethanol/water/potassium hydroxide, ethanol/water/sodium hydroxide, and ethanol/potassium hydroxide.

In another embodiment, the 6-mercaptopurine from the pharmaceutical composition comprising 6-mercaptopurine was spray granulated from a solution onto an acceptable pharmaceutical carrier powder wherein the solvent for the solution of 6-mercaptopurine comprises ethanol/potassium hydroxide or ethanol/water/potassium hydroxide.

In another embodiment, the 6-mercaptopurine from the pharmaceutical composition comprising 6-mercaptopurine was spray granulated from a solution onto an acceptable pharmaceutical carrier powder wherein the pharmaceutical carrier powder comprises a powder selected from the group consisting of lactose, starch, microcrystalline cellulose, calcium phosphate, powdered cellulose, sorbitol, and sucrose.

In another embodiment, the 6-mercaptopurine from the pharmaceutical composition comprising 6-mercaptopurine was spray granulated from a solution onto a pharmaceutical carrier powder that comprises lactose or microcrystalline cellulose.

In another embodiment, the 6-mercaptopurine from the pharmaceutical composition comprising 6-mercaptopurine was spray granulated from a solution onto an acceptable pharmaceutical carrier powder wherein the pharmaceutical carrier powder was pre-sprayed with a solution of a pharmaceutically acceptable acid in a molar amount that is greater than the molar amount of potassium hydroxide or other pharmaceutically acceptable base in the 6-mercaptopurine solution applied to the pharmaceutical carrier powder.

In another embodiment, the 6-mercaptopurine from the pharmaceutical composition comprising 6-mercaptopurine was spray granulated from a solution onto an acceptable pharmaceutical carrier powder wherein the pharmaceutical carrier powder was pre-sprayed with a solution comprising an acid selected from the group consisting of acetic acid, ascorbic acid, benzoic acid, citric acid, and tartaric acid.

In another embodiment, the 6-mercaptopurine from the pharmaceutical composition comprising 6-mercaptopurine was spray granulated from a solution onto an acceptable pharmaceutical carrier powder wherein the pharmaceutical carrier powder was pre-sprayed with a solution of citric acid.

As described herein, the a pharmaceutical compositions may comprise about 3% to about 20% of 6-mercaptopurine and about 4% to about 30% of potassium citrate.

As described herein, the a pharmaceutical compositions may comprise about 8% 6-mercaptopurine and about 5% potassium citrate.

As described herein, the a pharmaceutical compositions may comprise about 3% to about 20% of 6-mercaptopurine wherein the 6-mercaptopurine was spray granulated from solution onto an acceptable pharmaceutical carrier powder wherein the pharmaceutical carrier powder was pre-sprayed with a solution of citric acid.

In another embodiment, the method of making the pharmaceutical composition of 6-mercaptopurine comprises the spray granulation of a solution of 6-mercaptopurine onto a pharmaceutical carrier wherein the 6-mercaptopurine is dissolved in a solvent that comprises a solvent selected from the group consisting of dimethylformamide, dimethylacetamide, dimethylsulfoxide, and mixtures thereof.

In another embodiment, method of making a pharmaceutical composition of 6-mercaptopurine comprises the spray granulation of a solution of 6-mercaptopurine onto a pharmaceutical carrier wherein the 6-mercaptopurine is dissolved in a solvent that comprises a solvent selected from the group consisting of water and an at least about stoichiometric amount of a pharmaceutically acceptable base, ethanol and an at least about stoichiometric amount of a pharmaceutically acceptable base, and ethanol/water mixtures and an at least about stoichiometric amount of a pharmaceutically acceptable base.

In another embodiment, the method of making a pharmaceutical composition of 6-mercaptopurine comprises the spray granulation of a solution of 6-mercaptopurine onto a pharmaceutical carrier wherein the 6-mercaptopurine is dissolved in a solvent that comprises a solvent selected from the group consisting of ethanol/water/potassium hydroxide, ethanol/water/sodium hydroxide, and ethanol/potassium hydroxide. In certain embodiments, the solvent consists essentially of ethanol/water/potassium hydroxide, ethanol/water/sodium hydroxide, or ethanol/potassium hydroxide.

In another embodiment, the method of making a pharmaceutical composition of 6-mercaptopurine comprises the spray granulation of a solution of 6-mercaptopurine onto a pharmaceutical carrier wherein the 6-mercaptopurine is dissolved in ethanol/potassium hydroxide, or ethanol/water/potassium hydroxide.

In another embodiment, the invention relates to a method of making a pharmaceutical composition of 6-mercaptopurine comprising the spray granulation of a solution of 6-mercaptopurine onto a pharmaceutical carrier wherein the pharmaceutical carrier comprises a powder selected from the group consisting of lactose, starch, microcrystalline cellulose, calcium phosphate, powdered cellulose, sorbitol, and sucrose, preferably lactose or microcrystalline cellulose.

In another embodiment, the invention relates to a method of making a pharmaceutical composition of 6-mercaptopurine comprising the spray granulation of a solution of 6-mercaptopurine onto a pharmaceutical carrier wherein the pharmaceutical carrier was pre-sprayed with a solution of a pharmaceutically acceptable acid in a molar amount that is greater than the molar amount of potassium hydroxide or other pharmaceutically acceptable base in the 6-mercaptopurine solution applied to the pharmaceutical carrier.

In another embodiment, the method of making a pharmaceutical composition of 6-mercaptopurine comprises the spray granulation of a solution of 6-mercaptopurine onto a pharmaceutical carrier using a fluidized bed granulator.

One embodiment of the invention is directed to 6-MP formulations that comprise 6-MP formulated into granulates by first dissolving the 6-MP in an organic solvent. Examples of solvents that can be used to dissolve the 6-MP to an extent sufficient to be able to apply the solution to a pharmaceutical powder for further processing are dimethylformamide, dimethylacetamide, and dimethylsulfoxide, or mixtures thereof. Lactose, starch, microcrystalline cellulose, calcium phosphate, powdered cellulose, sorbitol or sucrose are examples of pharmaceutically acceptable powders that can be used as powders for this granulation. Other pharmaceutical excipient powders are known in the art and may also be used. In a more preferred embodiment the organic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated, and a yet more preferred embodiment uses dimethylformamide to form the granulation solution. In a more preferred embodiment of the invention a lactose granulate is formed that comprises, on a weight/weight (w/w) basis, 1 to 35% 6-MP, more preferably 5 to 20% 6-MP, and most preferably about 13% 6-MP. These granulates are then mixed with other tablet excipients and formed into tablets comprising 0.5 mg to 150 mg of 6-MP for an approximate tablet weight of 500 mg with an about 50 mg dose the most preferred. Alternately the dose of 6-MP can be controlled by changing tablet weight using any of the preferred, more preferred, or most preferred granulates.

Tablets that comprise these formulations of 6-MP have improved dissolution properties. When testing these tablets in 900 ml of 0.1N HCl at 37°C in a USP apparatus II dissolution tester with paddles rotating at 50 rpm, the rate of dissolution is greatly enhanced compared to the standard formulation. The time to 50% of dissolution is below seven minutes more preferably below five minutes and exhibits a more than 30% reduction in the time to 50% dissolution, more preferably a more than 50% reduction in time to 50% dissolution, when compared to the standard formulation.

A more preferred embodiment of this invention is directed to 6-MP formulations that comprise 6-MP formulated into granulates by first dissolving the 6-MP in ethanol containing at least about a stoichiometric amount of base, water containing at least about a stoichiometric amount of base, or mixtures of ethanol/water containing at least about a stoichiometric amount of base. The base may be selected from any pharmaceutically acceptable base such as the hydroxide or carbonate salts of potassium, sodium, magnesium, ammonium, or calcium, with potassium hydroxide being preferred. Optionally, a binder such as polyvinylpyrrolidone (PVP) may be added to the solution. This basic solution of 6-MP is granulated onto a pharmaceutical carrier selected from the group of lactose, starch, microcrystalline cellulose, calcium phosphate, powdered cellulose, sorbitol, or sucrose. Other pharmaceutical excipient powders are known in the art and may also be used. In a preferred embodiment, the basic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated and a most preferred embodiment uses an ethanol/water solvent mixture and potassium hydroxide as the base. In another more preferred embodiment, microcrystalline cellulose is used as the pharmaceutical powder upon which the 6-MP is granulated, and a most preferred embodiment uses an ethanol/water solvent mixture and potassium hydroxide as the base. The basic granulate is neutralized with a slight stoichiometric excess of any pharmaceutically acceptable acid. Examples of such acids are acetic acid, ascorbic acid, benzoic acid, citric acid, and tartaric acid. In a more preferred embodiment the acid selected is citric acid. In a more preferred embodiment, the pharmaceutically acceptable acid is precoated in a slight stoichiometric excess onto the pharmaceutically acceptable carrier before it is used in the granulation with the basic organic solution of 6-MP. In a more preferred embodiment, the pharmaceutically acceptable carrier is lactose and the pharmaceutically acceptable acid that is preloaded in a slight stoichiometric excess is citric acid. A more preferred mode for applying the acid is spray granulation and a most preferred method uses a fluidized bed granulator. In a preferred embodiment of the invention a lactose granulate is formed that comprises 1 to 35% 6-MP, more preferably 5 to 20% 6-MP, and most preferably about 11% 6-MP. In another preferred embodiment of the invention, a microcrystalline cellulose granulate is formed that comprises 1 to 35% 6-MP, more preferably 5 to 20% 6-MP, and most preferably about 11% 6-MP. These granulates further comprise salts of pharmaceutically acceptable acids, more preferably the sodium or potassium salts of acetic acid, ascorbic acid, benzoic acid, citric acid, or tartaric acid and most preferably the potassium salt of citric acid. The potassium citrate is present in about a stoichiometric amount compared to the 6-MP. These granulates are then mixed with other tablet excipients and formed into tablets comprising 0.5 mg to 150 mg of 6-MP for an approximate total tablet weight of 650 mg with an about 50 mg dose of 6-MP being the most preferred. Alternately the dose of 6-MP can be controlled by changing tablet weight using any of the preferred, more preferred, or most preferred granulates. In another embodiment the final dosage form comprises about 3% to about 20% of 6-mercaptopurine and about 2% to about 30% of potassium citrate and more preferably about 5% to about 15% of 6-MP and about 2% to about 20% potassium citrate, and most preferably about 8% 6-mercaptopurine and about 5% potassium citrate.

Tablets that comprise these formulations of 6-MP have improved dissolution properties. When testing these tablets in 900 ml of 0.1N HCl at 37°C in a USP apparatus II dissolution tester with paddles rotating at 50 rpm, the rate of dissolution is greatly enhanced as compared to the standard formulation. The time to 50% of dissolution is below seven minutes, more preferably below five minutes, and exhibits a more than 30% reduction in the time to 50% dissolution, more preferably a more than 50% reduction in time to 50% dissolution, when compared to the standard formulation.

Another aspect of the invention is a method of producing compositions of 6-mercaptopurine which give improved rates of dissolution when tested in a dissolution bath. Standard formulation 6-MP tablets reach full dissolution after about an hour under acidic dissolution conditions using a USP type II dissolution unit with paddles rotating at 50 rpm. 50% dissolution is reached at between 10 and 15 minutes. Improved rates of dissolution are defined herein as a time to 50% of dissolution less than or equal to about seven minutes, more preferably less than or equal to about five minutes, or a more than 30% reduction in the time to 50% dissolution, more preferably a more than or equal to 50% reduction in the time to 50% dissolution, compared to the standard formulation. One aspect of the present invention is a method of forming 6-MP formulations that comprises granulating 6-MP into granulates by first dissolving the 6-MP in an organic solvent. Examples of solvents that can be used to dissolve the 6-MP to an extent sufficient to be able to apply the solution to a pharmaceutical powder for further processing are dimethylformamide, dimethylacetamide, and dimethylsulfoxide, or mixtures thereof. Lactose, starch, microcrystalline cellulose, calcium phosphate, powdered cellulose, sorbitol, or sucrose are examples of pharmaceutically acceptable powders that can be used as powders for this granulation. Other pharmaceutical excipient powders are known in the art and may also be used. In a more preferred embodiment the organic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated and a yet more preferred embodiment uses dimethylformamide to form the granulation solution. In a more preferred embodiment of the invention, a lactose granulate is formed that comprises 1 to 35% 6-MP, more preferably 5 to 20% 6-MP, and most preferably about 13% 6-MP. These granulates are then mixed with other tablet excipients and formed into tablets comprising 0.5 mg to 150 mg of 6-MP for an approximate tablet weight of 500 mg with an about 50 mg dose being the most preferred. Alternatively, the dose of 6-MP can be controlled by changing tablet weight using any of the preferred, more preferred, or most preferred granulates.

Tablets that comprise formulations of 6-MP made by this method have improved dissolution properties. When testing these tablets in 900 ml of 0.1N HCl at 37°C in a USP apparatus II dissolution tester with paddles rotating at 50 rpm, the rate of dissolution is greatly enhanced compared to the standard formulation. The time to 50% of dissolution is below seven minutes, more preferably below five minutes, and exhibits a more than 30% reduction in the time to 50% dissolution, more preferably a more than 50% reduction in time to 50% dissolution, when compared to the standard formulation.

A more preferred embodiment of this invention is a method of making 6-MP formulations that comprises granulating 6-MP into granulates by first dissolving the 6-MP in ethanol containing at least a stoichiometric amount of base, water containing at least a stoichiometric amount of base, or mixtures of ethanol/water containing at least a stoichiometric amount of base. The base may be selected from any pharmaceutically acceptable base such as the hydroxide or carbonate salts of potassium, sodium, magnesium, ammonium, or calcium, with potassium hydroxide being more preferred. Optionally, a binder such as polyvinylpyrrolidone (PVP) may be added to the solution. This basic solution of 6-MP is granulated onto a pharmaceutical carrier selected from the group consisting of lactose, starch, microcrystalline cellulose, calcium phosphate, powdered cellulose, sorbitol, and sucrose. Other pharmaceutical excipient powders are known in the art and may also be used. In a more preferred embodiment the basic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated, and a most preferred embodiment uses an ethanol/water solvent mixture and potassium hydroxide as the base. In another more preferred embodiment, microcrystalline cellulose is used as the pharmaceutical powder upon which the 6-MP is granulated, and a most preferred embodiment uses an ethanol/water solvent mixture and potassium hydroxide as the base. The basic granulate is neutralized with a stoichiometric excess of any pharmaceutically acceptable acid. Examples of such acids are acetic acid, ascorbic acid, benzoic acid, citric acid, and tartaric acid. In a more preferred embodiment, the acid selected is citric acid. In a more preferred embodiment, the pharmaceutically acceptable acid is preloaded in a slight stoichiometric excess onto the pharmaceutically acceptable carrier before it is used in the granulation with the basic organic solution of 6-MP. In a more preferred embodiment, the pharmaceutically acceptable carrier is lactose and the pharmaceutically acceptable acid that is preloaded in a slight stoichiometric excess is citric acid. A more preferred method for applying the acid is spray granulation, and a most preferred method uses a fluidized bed granulator. In a preferred embodiment of the invention, a lactose granulate is formed that comprises 1 to 35% 6-MP, preferably 5 to 20% 6-MP, and most preferably about 11% 6-MP. In another preferred embodiment of the invention, a microcrystalline cellulose granulate is formed that comprises 1 to 35% 6-MP, more preferably 5 to 20% 6-MP, and most preferably about 11% 6-MP. These granulates further comprise salts of pharmaceutically acceptable acids, preferably the sodium or potassium salts of acetic acid, ascorbic acid, benzoic acid, citric acid, or tartaric acid, and most preferably the potassium salt of citric acid. The potassium citrate is present in about a stoichiometric amount compared to the 6- MP. These granulates are then mixed with other tablet excipients and formed into tablets comprising 0.5 mg to 150 mg of 6-MP for an approximate total tablet weight of 650 mg, with an about 50 mg dose of 6-MP in the tablet being most preferred. Alternatively, the dose of 6-MP can be controlled by changing tablet weight using any of the preferred, more preferred, or most preferred granulates. In another embodiment, the final dosage form comprises about 3% to about 20% of 6-mercaptopurine and about 2% to about 30% of potassium citrate, preferably about 5% to about 15% of 6-MP and about 2% to about 20% potassium citrate, and most preferably about 8% 6-mercaptopurine and about 5% potassium citrate.

Tablets that comprise formulations of 6-MP made by this method have improved dissolution properties. When testing these tablets in 900 ml of 0.1N HCl at 37°C in a USP apparatus II dissolution tester with paddles rotating at 50 rpm, the rate of dissolution is greatly enhanced compared to the standard formulation. The time to 50% of dissolution is below seven minutes, preferably below five minutes, and exhibits a more than 30% reduction in the time to 50% dissolution, preferably a more than 50% reduction in time to 50% dissolution, when compared to the standard formulation.

Also described herein is a method of producing compositions of 6-mercaptopurine which provide enhanced bioavailability compared to the standard formulation. The enhanced bioavailability may be a rise in average AUCt or AUC_{I} of about 5% or more, preferably a rise of about 15% or more, and most preferably a rise of 20% or more. Alternatively, the average ratio of the individual AUCt values for the test and reference formulations is about 1.05 or more, preferably 1.15 or more, and most preferably 1.20 or more. One embodiment of this aspect of the invention is a method of making 6-MP formulations that comprises granulating 6-MP into granulates by first dissolving the 6-MP in an organic solvent. Examples of solvents that can be used to dissolve the 6-MP to an extent sufficient to be able to apply the solution to a pharmaceutical powder for further processing are dimethylformamide, dimethylacetamide, and dimethylsulfoxide, or mixtures thereof. Lactose, starch, microcrystalline cellulose, calcium phosphate, powdered cellulose, sorbitol, or sucrose are examples of pharmaceutically acceptable powders that can be used as powders for this granulation. Other pharmaceutical excipient powders are known in the art and may also be used. In a more preferred embodiment, the organic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated, and a yet more preferred embodiment uses dimethylformamide to form the granulation solution. In a more preferred embodiment of the invention, a lactose granulate is formed that comprises 1 to 35% 6-MP, preferably 5 to 20% 6-MP, and most preferably about 13% 6-MP. These granulates are then mixed with other tablet excipients and formed into tablets comprising 0.5 mg to 150 mg of 6-MP for an approximate total tablet weight of 500 mg, with an about 50 mg of 6-MP in that tablet being the dose most preferred. Alternatively, the dose of 6-MP can be controlled by changing tablet weight using any of the preferred, more preferred, or most preferred granulates.

Also described herein is a method of producing 6-MP formulations that comprises granulating 6-MP into granulates by first dissolving the 6-MP in ethanol containing at least about a stoichiometric amount of base, water containing at least about a stoichiometric amount of base, or mixtures of ethanol/water containing at least about a stoichiometric amount of base. The base may be selected from any pharmaceutically acceptable base such as the hydroxide or carbonate salts of potassium, sodium, magnesium, ammonium, or calcium, with potassium hydroxide being more preferred. Optionally, a binder such as polyvinylpyrrolidone (PVP) may be added to the solution. This basic solution of 6-MP is granulated onto a pharmaceutical carrier selected from the group of lactose, starch, microcrystalline cellulose, calcium phosphate, powdered cellulose, sorbitol, and sucrose. Other pharmaceutical excipient powders are known in the art and may also be used. In a more preferred embodiment the basic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated, and a most preferred embodiment uses an ethanol/water solvent mixture and potassium hydroxide as the base. In another more preferred embodiment, microcrystalline cellulose is used as the pharmaceutical powder upon which the 6-MP is granulated, and a most preferred embodiment uses an ethanol/water solvent mixture and potassium hydroxide as the base. The basic granulate is neutralized with a slight stoichiometric excess of any pharmaceutically acceptable acid. Examples of such acids are acetic acid, ascorbic acid, benzoic acid, citric acid, and tartaric acid. In a more preferred embodiment, the acid selected is citric acid. In a more preferred embodiment, the pharmaceutically acceptable acid is preloaded in a slight stoichiometric excess onto the pharmaceutically acceptable carrier before it is used in the granulation with the basic organic solution of 6-MP. In a more preferred embodiment, the pharmaceutically acceptable carrier is lactose and the pharmaceutically acceptable acid that is preloaded in an about slight stoichiometric excess is citric acid. A more preferred mode for applying the acid is spray granulation and a most preferred method uses a fluidized bed granulator. In a preferred embodiment of the invention a lactose granulate is formed that comprises 1 to 35% 6-MP, preferably 5 to 20% 6-MP, and most preferably about 11% 6-MP. In another preferred embodiment of the invention, a microcrystalline cellulose granulate is formed that comprises 1 to 35% 6-MP, more preferably 5 to 20% 6-MP, and most preferably about 11% 6-MP. These granulates further comprise salts of pharmaceutically acceptable acids, preferably the sodium or potassium salts of acetic acid, ascorbic acid, benzoic acid, citric acid, or tartaric acid, and most preferably the potassium salt of citric acid. The potassium citrate is present in about a stoichiometric amount compared to the 6- MP. These granulates are then mixed with other tablet excipients and formed into tablets comprising 0.5 mg to 150 mg of 6-MP for an approximate total tablet weight of 650 mg, with an about 50 mg dose of 6-MP in the tablet being preferred. Alternatively, the dose of 6-MP can be controlled by changing tablet weight using any of the preferred, more preferred, or most preferred granulates. In another embodiment the final dosage form comprises about 3% to about 20% of 6-mercaptopurine and about 2% to about 30% of potassium citrate, preferably about 5% to about 15% of 6-MP and about 2% to about 20% potassium citrate, and most preferably about 8% 6-mercaptopurine and about 5% potassium citrate.

Tablets that comprise formulations of 6-MP made by this method have improved dissolution properties and improved bioavailability, by more than 5%, preferably by more than 15%, and most preferably by more than 20%, when tested in beagle dogs.

Also described herein is a method of treating patients in need of treatment with 6-MP by dosing them with formulations of 6-MP that have enhanced bioavailability compared to the standard formulation. Examples of patients in need of treatment with 6-MP are patients suffering from any disease in which a cytotoxic drug is beneficial such as leukemia, especially acute lymphocytic leukemia, or other cancers, as well as patients suffering from any disease for which an immunosuppressant drug is beneficial, such as Crohn's diseases, ulcerative colitis, or arthritis.

The enhanced bioavailability may be a rise in average AUCt or AUC_{I} of about 5% or more, preferably a rise of about 15% or more, and most preferably a rise of about 20% or more. Alternatively, the average ratio of the individual AUCt values for the test and reference formulations is about 1.05 or more, preferably 1.15 or more, and most preferably about 1.20 or more. One embodiment of this aspect of the invention is a method of dosing, to a mammal, 6-MP formulations that comprise granulates that were produced by first dissolving the 6-MP in an organic solvent. Examples of solvents that can be used to dissolve the 6-MP to an extent sufficient to be able to apply the solution to a pharmaceutical powder for further processing are dimethylformamide, dimethylacetamide, and dimethylsulfoxide, or mixtures thereof. Lactose, starch, microcrystalline cellulose, calcium phosphate, powdered cellulose, sorbitol, or sucrose are examples of pharmaceutically acceptable powders that can be used as powders for this granulation. Other pharmaceutical excipient powders are known in the art and may also be used. In a more preferred embodiment the organic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated and a yet more preferred embodiment uses dimethylformamide to form the granulation solution. In a more preferred embodiment of the invention a lactose granulate is formed that comprises 1 to 35% 6-MP, more preferably 5 to 20% 6-MP and most preferably about 13% 6-MP. These granulates are then mixed with other tablet excipients and formed into tablets comprising 0.5 mg to 150 mg of 6-MP for an approximate tablet weight of 500 mg with an about 50 mg dose the most preferred. Alternately the dose of 6-MP can be controlled by changing tablet weight using any of the preferred, more preferred, or most preferred granulates.

Other tablet excipients that may be used to formulate tablets comprising the pharmaceutical compositions of the present invention include binders, diluents, disintegrants, lubricants, colorants, and taste masking agents. Suitable binders include microcrystalline cellulose, modified celluloses, and povidone. Suitable diluents include calcium hydrogen phosphate (CaHPO₄), anhydrous; lactose; and mannitol. Suitable disintegrants include sodium starch glycollate (type A), sodium starch glycollate (type B), and crospovidone. Suitable lubricants include sodium stearyl fumarate, dimeticone, macrogol 6000, hydrogenated castor oil, and stearic acid.

Also described herein is a method of dosing, to a mammal, 6-MP formulations that comprise granulates that were produced by first dissolving the 6-MP in ethanol containing at least about a stoichiometric amount of base, water containing at least about a stoichiometric amout of base, or mixtures of ethanol/water containing at least about a stoichiometric amount of base. The base may be selected from any pharmaceutically acceptable base such as the hydroxide or carbonate salts of potassium, sodium, magnesium, ammonium, or calcium, with potassium hydroxide being preferred. Optionally, a binder such as polyvinylpyrrolidone (PVP) may be added to the solution. This basic solution of 6-MP is granulated onto a pharmaceutical carrier selected from the group of lactose, starch, microcrystalline cellulose, calcium phosphate, powdered cellulose, sorbitol and sucrose. Other pharmaceutical excipient powders are known in the art and may also be used. In a more preferred embodiment the basic solvent solution of 6-MP is spray granulated on to the powder so as to form a uniform coating. A preferred method of performing this spray granulation is by using a fluidized bed granulator. A more preferred embodiment uses lactose as the pharmaceutical powder upon which the 6-MP is granulated and a most preferred embodiment uses an ethanol/water solvent mixture and potassium hydroxide as the base. In another more preferred embodiment, microcrystalline cellulose is used as the pharmaceutical powder upon which the 6-MP is granulated, and a most preferred embodiment uses an ethanol/water solvent mixture and potassium hydroxide as the base. The basic granulate is neutralized with an about slight stoichiometric excess of any pharmaceutically acceptable acid. Examples of such acids are acetic acid, ascorbic acid, benzoic acid, citric acid, and tartaric acid. In a more preferred embodiment, the acid selected is citric acid. In a more preferred embodiment, the pharmaceutically acceptable acid is preloaded in a slight stoichiometric excess onto the pharmaceutically acceptable carrier before it is used in the granulation with the basic organic solution of 6-MP. In a more preferred embodiment the pharmaceutically acceptable carrier is lactose and the pharmaceutically acceptable acid that is preloaded in a slight stoichiometric excess is citric acid. A more preferred mode for applying the acid is spray granulation and a most preferred method uses a fluidized bed granulator. In a preferred embodiment of the invention, a lactose granulate is formed that comprises 1 to 35% 6-MP, more preferably 5 to 20% 6-MP, and most preferably about 11% 6-MP. In another preferred embodiment of the invention, a microcrystalline cellulose granulate is formed that comprises 1 to 35% 6-MP, more preferably 5 to 20% 6-MP, and most preferably about 11% 6-MP. These granulates further comprise salts of pharmaceutically acceptable acids, more preferably the sodium or potassium salts of acetic acid, ascorbic acid, benzoic acid, citric acid, or tartaric acid and most preferably the potassium salt of citric acid. The potassium citrate is present in about a stoichiometric amount compared to the 6-MP. These granulates are then mixed with other tablet excipients and formed into tablets comprising 0.5 mg to 150 mg of 6-MP for an approximate tablet weight of 650 mg, with an about 50 mg dose the most preferred. Alternatively, the dose of 6-MP can be controlled by changing tablet weight using any of the preferred, more preferred, or most preferred granulates. In another embodiment, the final dosage form comprises about 3% to about 20% of 6-mercaptopurine and about 2% to about 30% of potassium citrate and more preferably about 5% to about 15% of 6-MP and about 2% to about 20% potassium citrate, and most preferably about 8% 6-mercaptopurine and about 5% potassium citrate.

Also described herein is a composition for use in the treatment of patients in need thereof, wherein the patients in need of said treatment are treated with a dose similar to the dose given with the standard formulation, thereby achieving enhanced efficacy.

In an embodiment of the invention, the dose of treatment is lowered so as to have the same bioavailability as the standard treatment but achieved with a lower dose of drug. The result of the treatment is the same efficacy as the standard formulation with less exposure to potent drugs and an improved side effect profile.

Methods of making 6-mercaptopurine are known in the art. For example, 6-mercaptopurine can be made according to the processes described in G.H. Hitchings, G.B. Elion, U.S. Patent No. 2,697,702 or G.B. Elion , et al., J. Am. Chem. Soc. 74,411 (1952).

### EXAMPLES

### Example 1

### Mercaptopurine Spray Granulated from Dimethylformamide Solution

6-Mercaptopurine (6-MP, Orion-Fermion, 13.2 gm) was dissolved in dimethylformamide (DMF, Merck, 1.25 liter) with stirring over a period of 30 minutes. Lactose (DMV, 85 gm) was charged into a fluidized bed drier/granulator (FBD) and suspended by airflow. The air inlet temperature was 70°C. The DMF solution of 6-MP was sprayed into the suspended fluidized bed at a rate that maintained a bed temperature of 36°C. Total spraying time was 6 hours. The granulated lactose was subsequently dried in the FBD at 70°C for one hour and sieved through a 1.0 mm screen. The dry granulate (100 gm which contained 13.2 gm 6-MP) was mixed with potato starch (AVEBE, 25.9 grams), microcrystalline cellulose (Avicel 101, FMC, 13.2 grams) and croscarmellose sodium (Ac-Di-Sol,_FMC, 3.7 grams) for 8 minutes. Magnesium stearate (Brenntag, 0.5 grams) was added and the powder mixed for a further minute. The powder was pressed into tablets using a Korsch 106 rotary tablet press, using 12 mm flat faced round punches with the inscription ϕβ571. Final tablet weight was 542 mg and the 6-MP content was 50 mg (6-MP-IB batch 131-016-1).

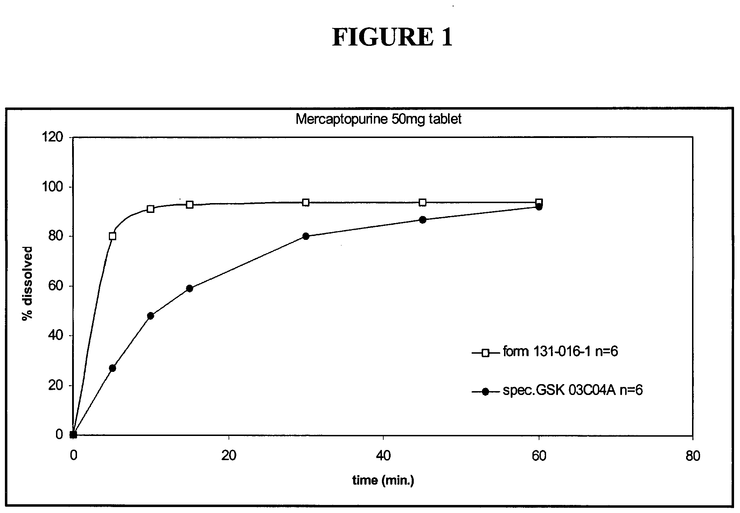

Dissolution analysis was carried out in a USP type II dissolution bath (VanKel) using 900 ml of 0.1N HCl kept at 37°C and stirred at 50 rpm. Samples were taken at 5, 10 15, 30, 45, and 60 minutes. PURINETHOL® (batch GSK03C04A) was tested under identical conditions. The 6-MP content of the samples was measured by UV spectroscopy at 325 nm against a standard curve. The results of the measurements are given in Table 1 and shown graphically in Figure 1.

**Table 1. Dissolution of 6-mercaptopurine from 6-MP-IB 131-016-1 vs. PURINETHOL® in 0.1N HCl**

| 6-MP-IB 131-016-1 | | PURINETHOL® GSK 03C04A | |
|---|---|---|---|
| Time (min) | Cumulative % | Time (min) | Cumulative % |
| 0 | 0 | 0 | 0 |
| 5 | 80 | 5 | 27 |
| 10 | 91 | 10 | 48 |
| 15 | 93 | 15 | 59 |
| 30 | 94 | 30 | 80 |
| 45 | 94 | 45 | 87 |
| 60 | 94 | 60 | 92 |

The results of the dissolution show that the DMF spray granulated 6-MP tablets give a much faster dissolution in 0.1N HCl than the standard formulation tablets. The time to 50% dissolution was better than halved with 80% being dissolved in 5 minutes and 91% at 10 minutes. The improved speed of dissolution of the product is expected to lead to improved bioavailability in vivo.

### Example 2

### Mercaptopurine Spray Granulated from Ethanol/Water/KOH Solution

Citric acid (Merck, 4.6 gm) was dissolved in 69 ml ethanol/water (70:30). This solution was sprayed onto a bed of lactose (DMV, 80 grams) suspended in an FBD granulator using the following conditions: inlet air temperature 55°C, bed temperature 28°C. 6-mercaptopurine (Orion-Fermion, 11.4 gm) was dissolved in 430 ml ethanol/water (80:20) containing pre-dissolved potassium hydroxide (Merck, 4.0 gram). The 6-MP solution was then sprayed onto the lactose/citric acid bed in the FBD using the following conditions: inlet air temperature 55°C, bed temperature 28°C. The bed was dried in situ at 55°C for 30 minutes. The dried granulate was passed through a 1.6 mm sieve. The dried and sieved granulate (100 grams) was mixed with potato starch (AVEBE, 26 grams), microcrystalline cellulose (Avicel 101, FMC, 11.4 grams), crospovidone (ISP Global Tech, 7.5 grams), and colloidal silicon dioxide (Degussa, 0.5 grams) for 8 minutes. Magnesium stearate (Brenntag, 2.2 gram) was added and the powder mixed for a further 2 minutes. The powder was pressed into tablets using a Korsch 106 rotary tablet press using 12 mm flat faced round punches with the inscription ϕβ571. Final tablet weight was 647 mg and the 6-MP content was 50 mg (6-MP-IB batch 131-018-6)

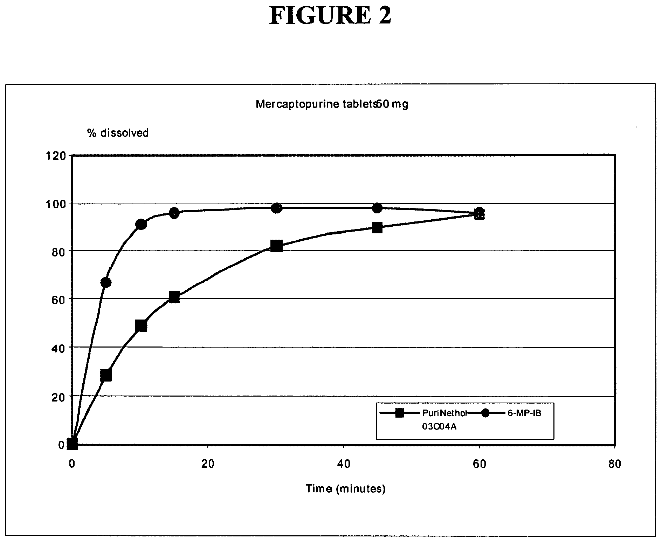

Dissolution analysis was carried out in a USP type II dissolution bath (VanKel) using 900 ml of 0.1N HCl kept at 37°C and stirred at 50 rpm. Samples were taken at 5, 10, 15, 30, 45, and 60 minutes. PURINETHOL® (batch GSK03CD4A) was tested under identical conditions. The 6-MP content of the samples was measured by UV spectroscopy at 325 nm against a standard curve. The results of the measurements are given in Table 2 and shown graphically in Figure 2.

**Table 2. Dissolution of 6-mercaptopurine from 6-MP-IB 131-018-6 vs. PURINETHOL® in 0.1N HCl**

| 6-MP-IB 131-018-6 | | PURINETHOL® GSK 03C04A | |
|---|---|---|---|
| Time (min) | Cumulative % | Time (min) | Cumulative % |
| 0 | 0 | 0 | 0 |
| 5 | 67 | 5 | 27 |
| 10 | 91 | 10 | 48 |
| 15 | 96 | 15 | 59 |
| 30 | 98 | 30 | 80 |
| 45 | 98 | 45 | 87 |
| 60 | 96 | 60 | 92 |

The results of the dissolution show that the basic ethanolic-water spray granulated 6-MP tablets give a much faster dissolution in 0.1N HCl than the standard formulation tablets. The time to 50% dissolution was better than halved with 67% being dissolved in 5 minutes and better than 90% at 10 minutes. The improved speed of dissolution of the product is expected to lead to improved bioavailability in vivo.

### Example 3

### Tablets of 6-MP Coated on Microcrystalline Cellulose or Lactose

This example present data from tablets in which 6-MP is coated on either microcrystalline cellulose or lactose. Table 3 shows a batch formula for tablets having 40 mg of 6-MP per tablet (the batch is for ∼ 1000 tablets), tablet weight 523 mg using 50% ethanol by volume (44.4% by weight) in both spraying steps.

**Table 3**

| | Raw material | (g) | (g) |
|---|---|---|---|
| 1 | Lactose monohydrate | 280 | ----- |
| 2 | Microcrystalline Cellulose | ----- | 280 |
| 3 | Citric Acid anhydrate | 19.5 | 19.5 |
| 4 | Alcohol denatured or USP | 96^{#} | 96^{#} |
| 5 | Purified Water | 120 | 120 |
| 6 | Mercaptopurine | 40.0 | 40.0 |
| 7 | Potassium hydroxide | 16.2 | 16.2 |
| 8 | PVP K30 | ----- | 10.4 |
| 9 | Alcohol denatured or USP | 600^{#} | 600^{#} |
| 10 | Purified Water | 750 | 750 |
| 11 | Colloidal Silicon Dioxide | 1.6 | 1.6 |
| 12 | Potato Starch | 24.4 | 24.4 |
| 13 | Crospovidone | 26.4 | 26.4 |
| 14 | Microcrystalline Cellulose | 91.6 | 91.6 |
| 15 | PVP K30 | 15.6 | 5.2 |
| 16 | Magnesium Stearate | 8.0 | 8.0 |

| | | | |
|---|---|---|---|
| # Density 0.8 g/mL | | | |

### Manufacturing method

### Solution A.

Mix alcohol (denatured or USP) (4) with purified water (5), add and dissolve citric acid (3).

### Coating step I (Aeromatic Strea 1)

Spray solution A on to lactose monohydrate (1) or microcrystalline cellulose (MCC) (2). Process parameters:

| | |
|---|---|
| Atomizing air: | 1 bar |
| Nozzle: | 1.0 mm |
| Inlet temperature: | 55°C |
| Exhaust temperature: | approx. 24°C |
| Spray rate: | approx. 9-10 g/min |
| Airflow rate: | approx. 54 m³/h |

### Solution B.

Mix alcohol (denatured or USP) (9) with purified water (10), add and dissolve potassium hydroxide (7). Add and dissolve 6-mercaptopurine (6). Optionally, PVP K30 (8) may be dissolved in this solution (either with lactose or with MCC-shown here with MCC).

### Coating step II (Aeromatic Strea 1)

Spray solution B onto the lactose monohydrate with citric acid or MCC with citric acid of coating step I .

### Process parameters:

| | |
|---|---|
| Atomizing air: | 1 bar |
| Nozzle: | 1.0 mm |
| Inlet temperature: | 55°C |
| Exhaust temperature: | approx. 24°C |
| Spray rate: | approx. 10-11 g/min |
| Airflow rate: | approx. 54-80 m³/h |

### Drying

Dry the lactose/citric acid/potassium hydroxide/6-mercaptopurine mixture or the MCC/citric acid/potassium hydroxide/PVP/6-mercaptopurine mixture.

### Process parameters:

| | |
|---|---|
| Inlet temperature: | 55°C |
| Exhaust temperature: | approx. 34°C |
| Airflow rate: | approx. 54-80 m³/h |

### Sieving I

Pass the lactose/citric acid/potassium hydroxide/6-mercaptopurine mixture or the MCC/citric acid/potassium hydroxide/PVP/6-mercaptopurine mixture through a 1.0 mm sieve.
Pass colloidal silicon dioxide (11) through a 1.0 mm sieve.

### Mixing I

Blend the lactose/citric acid/potassium hydroxide/6-mercaptopurine mixture or the MCC/citric acid/potassium hydroxide/PVP/6-mercaptopurine mixture with colloidal silicon dioxide for 2 minutes in a cubic tumbler.

### Sieving II

Pass potato starch (12), crospovidone (13), microcrystalline cellulose (14) and PVP K30 (15) through 1.0 mm sieve.

### Mixing II

Blend the lactose/citric acid/potassium hydroxide/6-mercaptopurine/colloidal silicon dioxide mixture or the MCC/citric acid/potassium hydroxide/PVP/6-mercaptopurine/colloidal silicon dioxide mixture with potato starch, crospovidone, microcrystalline cellulose and PVP K30 for 8 minutes in a cubic tumbler.

### Sieving III

Pass magnesium stearate (16) through a 1.0 mm sieve.

### Mixing III

Blend the mixture of Mixing step II with magnesium stearate for 2 minutes in a cubic tumbler.

### Tabletting

Compress the final mixture into tablets with tablet weight 523 mg (12mm, round convex R=9.5). Resistance to crushing of 5 -7 Kp, friability max. 1.0%, disintegration time < 5 min.

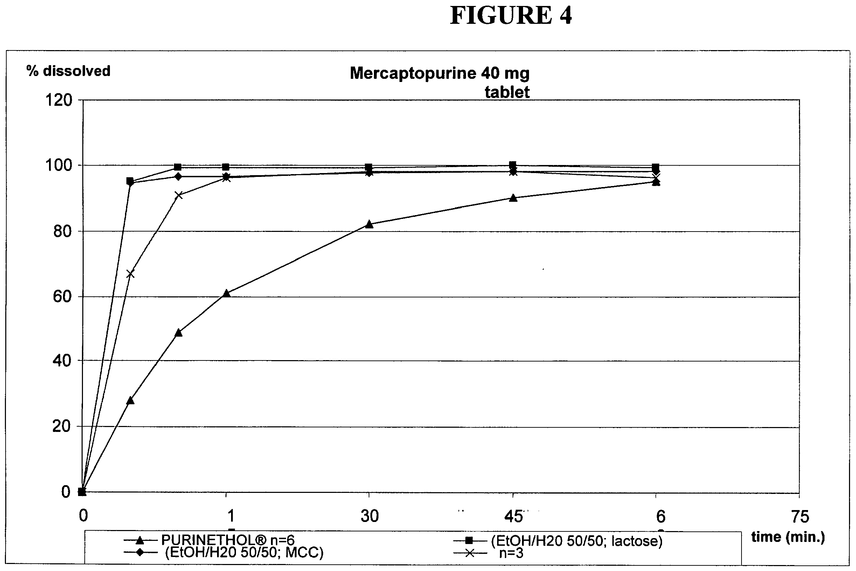

The results of the dissolution of 6- MP tablets prepared as in this example in 900 ml 0.1 N HCl at 37° C and 50 rpm is shown in Figure 4.

### Example 4

### A Comparative Bioavailability Study of a New Oral Formulation of 6-Mercaptopurine (6-MP-IB) vs. PURINETHOL® in Beagle Dogs

Study Objective- To determine the pharmacokinetic profile (AUCt and AUC_{I}, Cmax, Tmax, and half life of 6-mercaptopurine in the plasma following oral ingestion of each formulation to show improved bioavailability for 6-MP-IB
Study Design - Single center, single dose, non-randomized, open label (blinded to analyst), two treatment, two period crossover comparative bioavailability study.
Subjects - Six female beagle dogs, 2-3 years old, 9-11 kg body weight.

### Study administrations

1) PURINETHOL® (GSK): Half of a 50 mg tablet (i.e. 25 mg) of 6-mercaptopurine, Lot #A067350.
2) 6-MP-IB batch 131-018-6: Half of a 50 mg tablet (i.e. 25 mg) of 6-mercaptopurine.

The dogs received the half tablets in the fasted state (twelve hours fast). The tablets were placed in the back of the dog's throat. About 10 ml of water was squirted into the mouth with a syringe to facilitate swallowing. The mouth was examined to ensure that the tablet was swallowed.

### Blood collection and handling

Blood samples were taken from an indwelling catheter inserted in the jugular vein at 0 hour and at 0.25, 0.5, 1.0, 1.5, 2.0, 3.0, 4.0, 5.0, and 6.0 hours post dosing. Seven milliliters of blood was collected at each time point. The blood was chilled in ice immediately after collection. Within two minutes of collection the blood was transferred to tubes containing EDTA. The blood was processed to obtain the plasma within one hour. The plasma was stabilized with dithiothreitol and frozen to -80°C.

### Analyses

The analysis of 6-MP in the plasma was carried out at Anapharm Laboratories by a validated LC/MS/MS method.

### Study duration

Two study sessions with a wash out of two weeks between study sessions.

### Results

The results of the analysis of 6-MP in the plasma for all the dogs are given in Table 4A for the reference PURINETHOL® and in Table 4B for the test formulation 6-MP-IB.

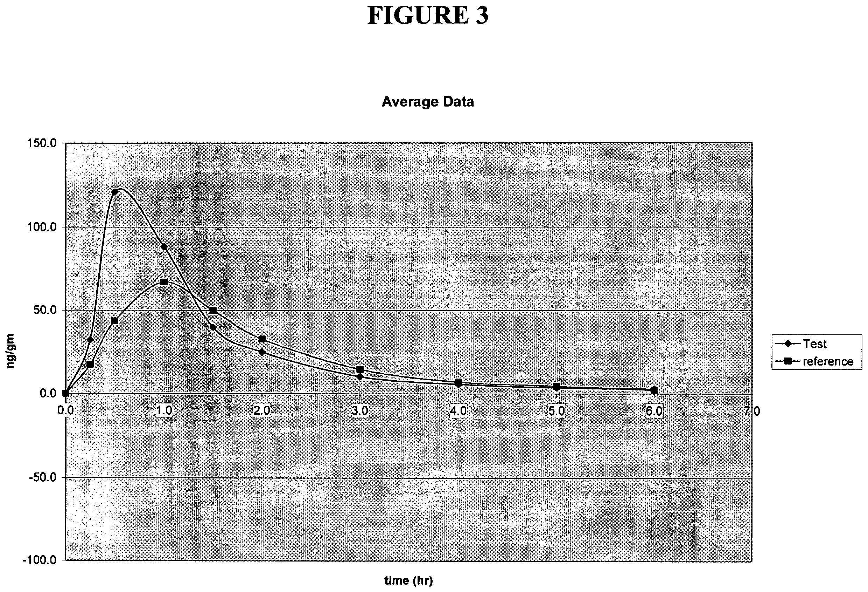

The results of the calculated pharmacokinetic parameters from the concentration data are collected in Table 5 while the results of a per dog ratio analysis are given in Table 6. The average pharmacokinetic profiles for all six dogs for each treatment are given in Figure 3.

One can see in Table 5 that the average AUCt and AUC_{I} are both about 20% higher for the test formulation (i.e., the composition of the present invention) when compared to the standard formulation. The Cmax is almost 70% higher. In the ratio analysis, shown in Table 6, where each dog is its own control, there is an average ratio of 1.26 or a 26% rise in the bioavailability of the test versus the reference product.

Figure 3 shows that the advantage of the faster dissolving formulation in bioavailability is in the early time points with higher drug concentrations being found shortly after drug ingestion. The Tmax for the averaged data is shorter for the test compared to reference despite the fact that the average Tmax (averaged over the individual dogs) is the same for the two formulations.

### Conclusions

The formulation provided by the present invention has been shown to give a more than 20% increase in bioavailability of 6-mercaptopurine in vivo when compared to an equivalent dose of the standard formulation. The improved bioavailability is expected to allow improved therapeutic outcomes.

**Table 4a. 6-mercaptopurine standard formulation (PURINETHOL®) concentrations (ng/ml)**

| Subject | Period | Draw Times (Hour) | | | | | | | | | |
|---|---|---|---|---|---|---|---|---|---|---|---|
| # | # | 0.000 | 0.250 | 0.500 | 1.00 | 1.50 | 2.00 | 3.00 | 4.00 | 5.00 | 6.00 |
| | | | | | | | | | | | |
| 02 | 1 | <2.00 | 35.15 | 38.98 | 149.72 | 131.27 | 80.36 | 26.90 | 11.01 | 7.87 | 5.37 |
| 03 | 1 | <2.00 | <2.00 | 53.24 | 41.64 | 31.96 | 39.83 | 19.10 | 8.85 | 4.76 | 2.73 |
| 04 | 1 | <2.00 | 21.69 | 112.90 | 54.94 | 26.45 | 15.24 | 9.75 | 12.12 | 8.24 | <2.00 |
| 05 | 1 | <2.00 | 20.97 | <2.00 | 123.11 | 75.23 | 62.88 | 41.19 | 13.16 | 8.96 | 4.87 |
| 06 | 1 | <2.00 | 61.09 | 143.83 | 106.22 | 42.88 | 22.53 | 8.98 | 5.84 | 3.23 | 2.19 |
| 11 | 1 | <2.00 | <2.00 | <2.00 | 59.72 | 91.79 | 39.99 | 10.20 | 4.53 | 2.46 | 2.03 |

**Table 4b. 6-mercaptopurine (6-MP-IB 131-018-6) concentrations (ng/ml)**

| Subject | Period | Draw Times (Hour) | | | | | | | | | |
|---|---|---|---|---|---|---|---|---|---|---|---|
| # | # | 0.000 | 0.250 | 0.500 | 1.00 | 1.50 | 2.00 | 3.00 | 4.00 | 5.00 | 6.00 |
| 02 | 2 | <2.00 | 25.07 | 109.97 | 181.60 | 77.10 | 37.32 | 15.22 | 8.52 | 5.29 | 3.83 |
| 03 | 2 | <2.00 | 129.92 | 159.49 | 79.27 | 77.05 | 37.12 | 11.66 | 6.64 | 3.62 | <2.00 |
| 04 | 2 | <2.00 | 30.68 | 173.75 | 99.24 | 35.45 | 21.17 | 8.88 | 4.35 | 2.71 | 8.29 |
| 05 | 2 | <2.00 | <2.00 | 380.69 | 172.31 | 59.78 | 27.99 | 20.85 | 12.50 | 8.26 | 5.91 |
| 06 | 2 | <2.00 | <2.00 | 4.61 | 104.99 | 44.09 | 53.45 | 19.34 | 10.30 | 6.69 | 4.05 |
| 11 | 2 | <2.00 | 70.75 | 139.59 | 69.21 | 24.87 | 21.03 | 5.47 | 3.15 | 2.14 | <2.00 |

**Table 5. Pharmokinetic results of dog study of 6-Mercaptopurine**

| Dog-session-treatment | AUCt (h^{∗}ng/g) | AUCi (h^{∗}ng/g) | t1/2 (h) | Tmax (h) | Cmax (ng/g) |
|---|---|---|---|---|---|
| 02 - 2- test | 235.8 | 241.7 | 1.1 | 1.0 | 181.6 |
| 03 - 2- test | 220.2 | 220.2 | 0.9 | 0.5 | 159.5 |
| 04 -2 - test | 176.1 | 188.2 | 1.0 | 0.5 | 173.8 |
| 05 - 2 - test | 324.4 | 338.5 | 1.7 | 1.0 | 380.7 |
| 06 - 2- test | 154.7 | 160.6 | 1.0 | 1.0 | 105.0 |
| 11 - 2- test | 143.6 | 143.6 | 0.9 | 0.5 | 139.6 |
| | | | | | |
| 02- 1- ref | 272.6 | 279.5 | 0.9 | 1.0 | 149.7 |
| 03 - 1- ref | 120.7 | 124.5 | 1.0 | 0.5 | 53.2 |
| 04 - 1-ref | 130.0 | 130.0 | 1.7 | 0.5 | 112.9 |
| 05 - 1- ref | 217.3 | 224.3 | 1.0 | 1.0 | 123.1 |
| 06 - 1 - ref | 179.8 | 183.3 | 1.1 | 0.5 | 143.8 |
| 11 - 1 - ref | 124.0 | 126.2 | 0.8 | 1.5 | 91.8 |
| | | | | | |
| **AVG(test)** | **209.1** | **215.5** | **1.1** | **0.8** | **190.0** |
| **AVG (ref)** | **174.1** | **178.0** | **1.1** | **0.8** | **112.4** |

**Table 6. Ratio Analysis**

| Dog | **Cmaxtest/Cmaxref** | **AUCt-test/AUCt-ref** |
|---|---|---|
| 02 | 1.21 | 0.86 |
| 03 | 3.00 | 1.82 |
| 04 | 1.54 | 1.35 |
| 05 | 3.09 | 1.49 |
| 06 | 0.73 | 0.86 |
| 11 | 1.52 | 1.16 |
| | | |
| **AVG** | **1.848** | **1.259** |

## Claims

1. A pharmaceutical composition comprising a pharmaceutical carrier powder, 6-mercaptopurine, a pharmaceutically acceptable base, and a pharmaceutically acceptable acid selected from the group consisting of acetic acid, ascorbic acid, citric acid, and tartaric acid, wherein the pharmaceutical composition exhibits enhanced solubility in aqueous acid as compared to the standard formulation as defined in the Physician's Desk Reference 57^{th} Edition, 2003, page 1615-1618 and wherein a solution of the pharmaceutically acceptable acid is sprayed onto the pharmaceutical carrier powder in a molar amount that is greater than the molar amount of pharmaceutically acceptable base and a solution of the 6-mercaptopurine and the pharmaceutically acceptable base is spray granulated onto the pharmaceutical carrier powder over the pharmaceutically acceptable acid.

2. The pharmaceutical composition of claim 1 wherein the spray granulation was carried out in a fluidized bed.

3. The pharmaceutical composition of claims 1 or 2 wherein the solution of the 6-mercaptopurine and the pharmaceutically acceptable base comprises:
a solvent selected from the group consisting of: water and an at least about stoichiometric amount of a pharmaceutically acceptable base, ethanol and an at least about stoichiometric amount of a pharmaceutically acceptable base, and ethanol/water mixtures and an at least about stoichiometric amount of a pharmaceutically acceptable base.

4. The pharmaceutical composition of any one of claims 1-3, wherein the solvent for the solution of the 6-mercaptopurine and the pharmaceutically acceptable base comprises ethanol/water/potassium hydroxide, ethanol/water/sodium hydroxide, or ethanol/potassium hydroxide.

5. A pharmaceutical composition comprising a pharmaceutical carrier powder and 6-mercaptopurine, wherein the pharmaceutical composition exhibits enhanced solubility in aqueous acid as compared to the standard formulation as defined in the Physician's Desk Reference 57^{th} Edition, 2003, page 1615-1618 and wherein a solution of the 6-mercaptopurine and an organic solvent is spray granulated onto the pharmaceutical carrier powder.

6. The pharmaceutical composition of claim 5, wherein the organic solvent is selected from the group consisting of dimethylformamide, dimethylacetamide, dimethylsulfoxide, and mixtures thereof.

7. The pharmaceutical composition of any one of claims 1-6 wherein the pharmaceutical carrier powder comprises a powder selected from the group consisting of: lactose, starch, microcrystalline cellulose, calcium phosphate, powdered cellulose, sorbitol, and sucrose, preferably the powder is lactose or microcrystalline cellulose.

8. The pharmaceutical composition of any one of claims 1-4 wherein the pharmaceutically acceptable acid is citric acid

9. A method of manufacturing a pharmaceutical composition comprising:
(a) spraying a solution of a pharmaceutically acceptable acid onto a pharmaceutical carrier;
(b) spray granulating the pharmaceutical carrier sprayed with a pharmaceutically acceptable acid produced in step (a) with a solution of 6-mercaptopurine and a pharmaceutically acceptable base, wherein the molar amount of the pharmaceutically acceptable acid is greater than the molar amount of the pharmaceutically acceptable base.

10. The method of claim 9 wherein the pharmaceutical carrier powder comprises a powder selected from the group consisting of: lactose, starch, microcrystalline cellulose, calcium phosphate, powdered cellulose, sorbitol, and sucrose, preferably the powder is lactose or microcrystalline cellulose, more preferably the powder is lactose, even more preferably the powder is microcrystalline cellulose.

11. A pharmaceutical composition as defined in any one of claims 1-8 for use in the treatment of Crohn's disease, which provides to the subject a therapeutic outcome with an improved side effect profile as compared to treating the subject with the standard formulation of 6-mercaptopurine as defined in the Physician's Desk Reference 57^{th} Edition, 2003, page 1615-1618, wherein the treatment comprises orally administering to the subject the pharmaceutical composition of any one of claims 1-8.

12. The pharmaceutical composition for use according to claim 11, wherein the pharmaceutical composition comprises 0.5 to 150 mg of 6-mercaptopurine, preferably the pharmaceutical composition comprises 50 mg of 6-mercaptopurine.

13. The pharmaceutical composition for use according to claims 11 or 12, wherein the pharmaceutical composition contains 15% less 6-mercaptopurine than the amount of 6-mercaptopurine in the standard formulation of 6-mercaptopurine as defined in the Physician's Desk Reference 57^{th} Edition, 2003, page 1615-1618.

14. The method of claims 9 or 10, wherein the pharmaceutically acceptable acid is selected from the group consisting of: acetic acid, ascorbic acid, citric acid and tartaric acid, preferably the pharmaceutically acceptable acid is citric acid.

15. The method of any one of claims 9, 10 or 14, wherein the pharmaceutically acceptable base is selected from the group consisting of: hydroxide or carbonate salts of potassium, sodium, magnesium, ammonium or calcium, preferably the pharmaceutically acceptable base is potassium hydroxide.

16. The method of any of claims 9, 10, 14 or 15, wherein the 6-mercaptopurine forms a coating on the solid carrier, preferably the 6-mercaptopurine forms a uniform coating on the solid carrier.

## Patentansprüche

1. Pharmazeutische Zusammensetzung, umfassend ein pharmazeutisches Träger-Pulver, 6-Mercaptopurin, eine pharmazeutisch akzeptable Base und eine pharmazeutisch akzeptable Säure, ausgewählt aus der Gruppe, bestehend aus Essigsäure, Ascorbinsäure, Zitronensäure und Weinsäure, wobei die pharmazeutische Zusammensetzung eine erhöhte Löslichkeit in wässriger Säure im Vergleich zu der Standardformulierung, wie in der Physician's Desk Reference 57. Ausgabe, 2003, Seite 1615-1618 definiert, aufweist und wobei eine Lösung der pharmazeutisch akzeptablen Säure auf das pharmazeutische Träger-Pulver in einer molaren Menge, die größer ist als die molare Menge an pharmazeutisch akzeptabler Base, aufgesprüht wird und eine Lösung des 6-Mercaptopurins und der pharmazeutisch akzeptablen Base auf das pharmazeutische Träger-Pulver über die pharmazeutisch akzeptable Säure sprühgranuliert wird.

2. Pharmazeutische Zusammensetzung nach Anspruch 1, wobei die Sprühgranulation in einem Fließbett durchgeführt wurde.

3. Pharmazeutische Zusammensetzung nach den Ansprüchen 1 oder 2, wobei die Lösung des 6-Mercaptopurins und der pharmazeutisch akzeptablen Base Folgendes umfasst:
ein Lösungsmittel, ausgewählt aus der Gruppe bestehend aus: Wasser und einer mindestens etwa stöchiometrischen Menge einer pharmazeutisch akzeptablen Base, Ethanol und einer mindestens etwa stöchiometrischen Menge einer pharmazeutisch akzeptablen Base und Ethanol/Wasser-Gemischen und mindestens einer etwa stöchiometrische Menge einer pharmazeutisch akzeptablen Base.

4. Pharmazeutische Zusammensetzung nach einem der Ansprüche 1-3, wobei das Lösungsmittel für die Lösung des 6-Mercaptopurins und der pharmazeutisch akzeptablen Base Ethanol/Wasser/Kaliumhydroxid, Ethanol/Wasser/Natriumhydroxid oder Ethanol/Kaliumhydroxid umfasst.

5. Pharmazeutische Zusammensetzung, umfassend ein pharmazeutisches Träger-Pulver und 6-Mercaptopurin, wobei die pharmazeutische Zusammensetzung eine erhöhte Löslichkeit in wässriger Säure im Vergleich zu der Standardformulierung, wie in der Physician's Desk Reference 57. Ausgabe, 2003, Seite 1615-1618 definiert, aufweist und wobei eine Lösung aus dem 6-Mercaptopurin und einem organischen Lösungsmittel auf das pharmazeutische Träger-Pulver sprühgranuliert wird.

6. Pharmazeutische Zusammensetzung nach Anspruch 5, wobei das organische Lösungsmittel aus der Gruppe ausgewählt ist, bestehend aus Dimethylformamid, Dimethylacetamid, Dimethylsulfoxid und deren Gemischen.

7. Pharmazeutische Zusammensetzung nach einem der Ansprüche 1-6, wobei das pharmazeutische Träger-Pulver ein Pulver umfasst, ausgewählt aus der Gruppe, bestehend aus: Lactose, Stärke, mikrokristalliner Cellulose, Calciumphosphat, pulverisierter Cellulose, Sorbit und Saccharose, wobei vorzugsweise das Pulver Lactose oder mikrokristalline Cellulose ist.

8. Pharmazeutische Zusammensetzung nach einem der Ansprüche 1-4, wobei die pharmazeutisch akzeptable Säure Zitronensäure ist.

9. Verfahren zur Herstellung einer pharmazeutischen Zusammensetzung, umfassend:
(a) Sprühen einer Lösung einer pharmazeutisch akzeptablen Säure auf einen pharmazeutischen Träger,
(b) Sprühgranulieren des in Schritt (a) hergestellten, mit einer pharmazeutisch akzeptablen Säure besprühten pharmazeutischen Trägers mit einer Lösung von 6-Mercaptopurin und einer pharmazeutisch akzeptablen Base, wobei die molare Menge der pharmazeutisch akzeptablen Säure größer ist als die molare Menge der pharmazeutisch akzeptablen Base.

10. Verfahren nach Anspruch 9, wobei das pharmazeutische Träger-Pulver ein Pulver umfasst, ausgewählt aus der Gruppe, bestehend aus: Lactose, Stärke, mikrokristalliner Cellulose, Calciumphosphat, pulverisierter Cellulose, Sorbit und Saccharose, wobei vorzugsweise das Pulver Lactose oder mikrokristalline Cellulose ist, wobei stärker bevorzugt das Pulver Lactose ist, noch stärker bevorzugt das Pulver mikrokristalline Cellulose ist.

11. Pharmazeutische Zusammensetzung nach einem der Ansprüche 1-8 zur Verwendung bei der Behandlung von Morbus Crohn, die dem Individuum ein Therapieergebnis mit einem verbesserten Nebenwirkungsprofil im Vergleich zu einer Behandlung des Individuums mit der Standardformulierung von 6-Mercaptopurin, wie in der Physician's Desk Reference 57. Ausgabe, 2003, Seite 1615-1618 definiert, bereitstellt, wobei die Behandlung das orale Verabreichen der pharmazeutischen Zusammensetzung nach einem der Ansprüche 1-8 an das Individuum umfasst.

12. Pharmazeutische Zusammensetzung zur Verwendung nach Anspruch 11, wobei die pharmazeutische Zusammensetzung 0,5 bis 150 mg 6-Mercaptopurin umfasst, wobei vorzugsweise die pharmazeutische Zusammensetzung 50 mg 6-Mercaptopurin umfasst.

13. Pharmazeutische Zusammensetzung zur Verwendung nach Anspruch 11 oder 12, wobei die pharmazeutische Zusammensetzung 15% weniger 6-Mercaptopurin umfasst als die Menge an 6-Mercaptopurin in der Standardformulierung von 6-Mercaptopurin, wie in der Physician's Desk Reference 57. Ausgabe, 2003, Seite 1615-1618 definiert, beträgt.

14. Verfahren nach den Ansprüchen 9 oder 10, wobei die pharmazeutisch akzeptable Säure aus der Gruppe ausgewählt ist, bestehend aus: Essigsäure, Ascorbinsäure, Zitronensäure und Weinsäure, wobei vorzugsweise die pharmazeutisch akzeptable Säure Zitronensäure ist.

15. Verfahren nach einem der Ansprüche 9, 10 oder 14, wobei die pharmazeutisch akzeptable Base aus der Gruppe ausgewählt ist, bestehend aus: Hydroxid oder Carbonatsalzen von Kalium, Natrium, Magnesium, Ammonium oder Calcium, wobei vorzugsweise die pharmazeutisch akzeptable Base Kaliumhydroxid ist.

16. Verfahren nach einem der Ansprüche 9, 10, 14 oder 15, wobei das 6-Mercaptopurin eine Beschichtung auf dem festen Träger bildet, wobei vorzugsweise das 6-Mercaptopurin eine gleichmäßige Beschichtung auf dem festen Träger bildet.

## Revendications

1. Composition pharmaceutique comprenant un véhicule pharmaceutique en poudre, de la 6-mercaptopurine, une base pharmaceutiquement acceptable et un acide pharmaceutiquement acceptable choisi dans le groupe constitué par l'acide acétique, l'acide ascorbique, l'acide citrique et l'acide tartrique, où la composition pharmaceutique présente une solubilité supérieure dans l'acide aqueux par rapport à la formule standard telle que définie dans Physician's Desk Reference 57^{e} édition, 2003, page 1615-1618 et où une solution de l'acide pharmaceutiquement acceptable est pulvérisée sur le véhicule pharmaceutique en poudre dans une quantité molaire qui est supérieure à la quantité molaire de la base pharmaceutiquement acceptable et une solution de la 6-mercaptopurine et de la base pharmaceutiquement acceptable est granulée par atomisation sur le véhicule pharmaceutique en poudre par-dessus l'acide pharmaceutiquement acceptable.

2. Composition pharmaceutique selon la revendication 1, où la granulation par atomisation est mise en oeuvre en lit fluidisé.

3. Composition pharmaceutique selon les revendications 1 ou 2, où la solution de la 6-mercaptopurine et de la base pharmaceutiquement acceptable comprend :
un solvant choisi dans le groupe constitué par les suivants : eau et au moins une quantité approximativement stoechiométrique d'une base pharmaceutiquement acceptable, éthanol et au moins une quantité approximativement stoechiométrique d'une base pharmaceutiquement acceptable, et mélanges éthanol/eau et au moins une quantité approximativement stoechiométrique d'une base pharmaceutiquement acceptable.

4. Composition pharmaceutique selon l'une quelconque des revendications 1 à 3, où le solvant de la solution de la 6-mercaptopurine et de la base pharmaceutiquement acceptable comprend éthanol/eau/hydroxyde de potassium, éthanol/eau/hydroxyde de sodium ou éthanol/hydroxyde de potassium.

5. Composition pharmaceutique comprenant un véhicule pharmaceutique en poudre et de la 6-mercaptopurine, où la composition pharmaceutique présente une solubilité supérieure dans l'acide aqueux par rapport à la formule standard telle que définie dans Physician's Desk Reference 57^{e} édition, 2003, page 1615-1618 et où une solution de la 6-mercaptopurine et d'un solvant organique est granulée par atomisation sur le véhicule pharmaceutique en poudre.

6. Composition pharmaceutique selon la revendication 5, où le solvant organique est choisi dans le groupe constitué par le diméthylformamide, le diméthylacétamide, le diméthylsulfoxyde et leurs mélanges.

7. Composition pharmaceutique selon l'une quelconque des revendications 1 à 6, où le véhicule pharmaceutique en poudre comprend une poudre choisie dans le groupe constitué par les suivantes : lactose, amidon, cellulose microcristalline, phosphate de calcium, cellulose en poudre, sorbitol et sucrose, préférentiellement, la poudre est du lactose ou de la cellulose microcristalline.

8. Composition pharmaceutique selon l'une quelconque des revendications 1 à 4, où l'acide pharmaceutiquement acceptable est l'acide citrique.

9. Procédé de fabrication d'une composition pharmaceutique comprenant :
(a) la pulvérisation d'une solution d'un acide pharmaceutiquement acceptable sur un véhicule pharmaceutique ;
(b) la granulation par atomisation du véhicule pharmaceutique pulvérisé avec un acide pharmaceutiquement acceptable produit dans l'étape (a) avec une solution de 6-mercaptopurine et d'une base pharmaceutiquement acceptable, où la quantité molaire de l'acide pharmaceutiquement acceptable est supérieure à la quantité molaire de la base pharmaceutiquement acceptable.

10. Procédé selon la revendication 9, où le véhicule pharmaceutique en poudre comprend une poudre choisie dans le groupe constitué par les suivantes : lactose, amidon, cellulose microcristalline, phosphate de calcium, cellulose en poudre, sorbitol et sucrose, préférentiellement, la poudre est le lactose ou la cellulose microcristalline, plus préférentiellement, la poudre est le lactose, encore plus préférentiellement, la poudre est la cellulose microcristalline.

11. Composition pharmaceutique selon l'une quelconque des revendications 1 à 8 pour utilisation dans le traitement de la maladie de Crohn, qui donne au sujet un résultat thérapeutique dont le profil d'effets secondaires est amélioré par rapport au traitement du sujet par la formule standard de 6-mercaptopurine telle que définie dans Physician's Desk Reference 57^{e} édition, 2003, page 1615-1618, où le traitement comprend l'administration orale au sujet de la composition pharmaceutique selon l'une quelconque des revendications 1 à 8.

12. Composition pharmaceutique pour utilisation selon la revendication 11, où la composition pharmaceutique comprend 0,5 à 150 mg de 6-mercaptopurine, préférentiellement, la composition pharmaceutique comprend 50 mg de 6-mercaptopurine.

13. Composition pharmaceutique pour utilisation selon les revendications 11 ou 12, où la composition pharmaceutique contient 15 % de 6-mercaptopurine de moins que la quantité de 6-mercaptopurine dans la formule standard de 6-mercaptopurine telle que définie dans Physician's Desk Reference 57^{e} édition, 2003, page 1615-1618.

14. Procédé selon les revendications 9 ou 10, où l'acide pharmaceutiquement acceptable est choisi dans le groupe constitué par les suivants : acide acétique, acide ascorbique, acide citrique et acide tartrique, préférentiellement l'acide pharmaceutiquement acceptable est l'acide citrique.

15. Procédé selon l'une quelconque des revendications 9, 10 ou 14, où la base pharmaceutiquement acceptable est choisie dans le groupe constitué par les suivants : sels d'hydroxyde ou de carbonate de potassium, de sodium, de magnésium, d'ammonium ou de calcium, préférentiellement, la base pharmaceutiquement acceptable est l'hydroxyde de potassium.

16. Procédé selon l'une quelconque des revendications 9, 10, 14 ou 15, où la 6-mercaptopurine forme un revêtement sur le véhicule solide, préférentiellement, la 6-mercaptopurine forme un revêtement uniforme sur le véhicule solide.
